# EUROPÄISCHE PATENTSCHRIFT

(11) **EP 3 115 074 B1**
(45) Veröffentlichungstag und Bekanntmachung des Hinweises auf die Patenterteilung: **26.12.2018**
(21) Anmeldenummer: 16001485.8
(22) Anmeldetag: 05.07.2016
(51) Int. Cl.: A61M 16/00

(54) **BEATMUNGSGERÄT MIT ZWEI UNTERSCHIEDLICHEN BETRIEBSWEISEN**
VENTILATOR HAVING TWO DIFFERENT OPERATIONAL CONFIGURATIONS
RESPIRATEUR ARTIFICIEL COMPRENANT DEUX MODES DE FONCTIONNEMENT DIFFERENTS

(30) Priorität: 06.07.2015 DE 102015008524
(43) Veröffentlichungstag der Anmeldung: 11.01.2017
(73) Patentinhaber: Löwenstein Medical Technology S.A., 2557 Luxembourg (LU)
(72) Erfinder: Göbel, Christof, 22457 Hamburg (DE)
(74) Vertreter: Marx, Thomas

(56) Entgegenhaltungen:
- EP-A1- 1 972 356
- WO-A1-2010/070498
- US-A1- 2001 004 893
- US-A1- 2009 229 611
- US-A1- 2011 041 850
- US-A1- 2012 145 152
- US-A1- 2014 171 818
- US-B1- 6 629 527

## Beschreibung

Die vorliegende Erfindung betrifft ein Beatmungsgerät mit einer ersten Betriebsweise und einer zweiten Betriebsweise, die ein geringeres Maß an Unterstützung der Atemarbeit, bereitstellt als die erste Betriebsweise.

Bei der langfristigen, klinischen und außerklinischen Beatmung wird die körpereigene Atemmuskulatur durch maschinelle Unterstützung entlastet. Die maschinelle Unterstützung findet statt entweder mit Druck- oder mit Volumenvorgabe in den Einatemphasen, während die Ausatmung passiv abläuft.

Der Körper gewöhnt sich mit zunehmender Beatmungsdauer (ab etwa einer Woche) allerdings daran, die Atemmuskulatur nicht selbst einzusetzen, wodurch sie geschwächt wird (Muskelatrophie). Die Schwächung kann nach einiger Zeit so ausgeprägt sein, dass der Patient nicht mehr in der Lage ist, von selbst eine ausreichende Atemleistung zu erzeugen.

Eine ärztlich eingestellte und kontrollierte Entwöhnung des Patienten von der Beatmung ist dann notwendig, um den Patienten allmählich in die Lage zu versetzen, auf die künstliche Beatmung zu verzichten.

Die EP1972356 A1 beschreibt die Entwöhnung von der klinischen Beatmung. Die EP1972356 A1 schlägt dazu vor, die Bereitschaft des Patienten für die Entwöhnung von der klinischen Beatmung zu bestimmen. Dazu wird der Sauerstoffverbrauch des Patienten bestimmt, nachdem die Unterstützung durch das Beatmungsgerät abgesenkt wurde. In einem Ausführungsbeispiel wird die Unterstützung durch das Beatmungsgerät dadurch abgesenkt, dass der inspiratorische Druck (IPAP) abgesenkt wird.

Die US2012145152 A1 beschreibt ebenfalls die Entwöhnung von der klinischen Beatmung. Die US2012145152 A1 schlägt als eine Möglichkeit der Entwöhnung vor, den Druck anzupassen und insbesondere den PEEP abzusenken.

Die WO2010070498 A1, US2011041850 A1, US2014171818 A1, US2001004893 A1, US2009229611 A1 offenbaren alternative Verfahren der Entwöhnung von der klinischen Beatmung.
Bei der kurzfristigen oder intermittierenden Beatmung tritt eine solche Schwächung der Atemmuskulatur nicht oder nur bedingt ein. Eine ärztlich kontrollierte Entwöhnung des Patienten von der Beatmung ist daher nicht notwendig. Trotzdem berichten Patienten von Problemen bei dem Wechsel von der Beatmung zur selbständigen Atmung. Werden Patienten beispielsweise über Nacht beatmet, so beendet das Ablegen der Maske am Morgen die Atemunterstützung abrupt. Der Patient muss seine Atemmuskulatur unmittelbar aktivieren und die vollständige Atemarbeit übernehmen. Dieses wird von Patienten als unangenehm oder auch als starke Belastung empfunden.
Aus diesem Grund wäre ein angenehmer Übergang von der Beatmung zur selbständigen Atmung wünschenswert.
Die vorliegende Erfindung betrifft vorzugsweise - aber nicht ausschließlich - Beatmungsformen mit Druckvorgabe. Die Unterstützung oder Übernahme von Atemarbeit seitens des Beatmungsgerätes wird wesentlich bestimmt durch die Differenz der Druckniveaus zwischen der Ein- und Ausatmung (IPAP-EPAP oder inspiratorische Druckunterstützung). Erfindungsgemäß wird im Folgenden von einer 'hohen Unterstützung' der Atemarbeit in dem Sinne gesprochen, wenn das Beatmungsgerät die volle - vom Arzt eingestellte und damit therapeutisch angestrebte - inspiratorische Druckunterstützung bereitstellt.

Aufgabe der vorliegenden Erfindung ist es daher ein Beatmungsgerät anzugeben, welches dem Patienten einen selbstbestimmten und angenehmen Übergang von der Beatmung zur selbständigen Atmung ermöglicht.
Die Aufgabe wird erfindungsgemäß gelöst durch ein Beatmungsgerät gemäß Anspruch 1, mit einer Steuereinheit die eine erste Betriebsweise, mit einem hohen Maß an Unterstützung der Atemarbeit, bereitstellt, wobei die Steuereinheit nach Aktivierung über ein Auswahlmittel, eine zweite Betriebsweise, mit einem geringeren Maß an Unterstützung der Atemarbeit, bereitstellt, wobei die Steuereinheit in der zweiten Betriebsweise den IPAP schneller absenkt als den EPAP.
Das Auswahlmittel ist beispielsweise ein Schalter am Beatmungsgerät, oder eine Menüsteuerung am Gerät oder ein Touchscreen am Gerät oder eine Fernsteuerung über Kabel oder drahtlos (Bluetooth).

Erfindungsgemäß ist alternativ oder ergänzend auch vorgesehen, dass die einzelnen Schritte (Phasen) der zweiten Betreibsweise sich jeweils über eine definierte Zeit erstrecken.

Erfindungsgemäß ist alternativ oder ergänzend auch vorgesehen, dass die einzelnen Schritte (Phasen) sich jeweils über eine definierte Anzahl von Atemzügen erstrecken.

Erfindungsgemäß ist alternativ oder ergänzend auch vorgesehen, dass die einzelnen Schritte (Phasen) basierend auf Anwendereingaben erfolgen.

Erfindungsgemäß ist alternativ oder ergänzend auch vorgesehen, dass während der zweiten Betriebsweise der Wechsel zwischen Phasen mit unterschiedlicher Unterstützung der Atemarbeit auf Signalen der Beatmung beruht.

Erfindungsgemäß ist alternativ oder ergänzend auch vorgesehen, dass während der zweiten Betriebsweise der Wechsel zwischen Phasen mit unterschiedlicher Unterstützung der Atemarbeit auf Signalen einer Pulsoxymetrie beruht. Dabei kann das Pulsoxymetrie-Gerät mit dem Beatmungsgerät gekoppelt sein und die Daten der Pulsoxymetrie können direkt von der Steuereinheit des Beatmungsgerätes berücksichtigt werden.

Erfindungsgemäß ist alternativ oder ergänzend auch vorgesehen, dass während der zweiten Betriebsweise der Wechsel zwischen Phasen mit unterschiedlicher Unterstützung der Atemarbeit auf CO2-Signalen beruht. Dabei kann das Gerät zur Erfassung des CO2 mit dem Beatmungsgerät gekoppelt sein und die Daten der CO2-Ermittlung können direkt von der Steuereinheit des Beatmungsgerätes berücksichtigt werden. Die CO2-Ermittlung kann in der Ausatemluft und/oder transkutan und/oder invasiv erfolgen.

Erfindungsgemäß ist alternativ oder ergänzend auch vorgesehen, dass die Steuereinheit die zweite Betriebsweise mit einem schrittweisen Absenken des IPAP, gegenüber dem Niveau der erste Betriebsweise, bereitstellt.

Beispielsweise ist alternativ oder ergänzend auch vorgesehen, dass die Steuereinheit die zweite Betriebsweise mit einem zeitweisen Absenken und Wiederansteigen des IPAP bereitstellt.

Beispielsweise ist alternativ oder ergänzend auch vorgesehen, dass die Steuereinheit die zweite Betriebsweise mit zeitweisen Phasen konstanten Druckes (CPAP-Phasen) bereitstellt.

Erfindungsgemäß ist alternativ oder ergänzend auch vorgesehen, dass die Steuereinheit die zweite Betriebsweise mit zumindest, gegenüber der erste Betriebsweise, zeitweise verkürzter Inspirationszeit (Ti2, Ti3) bereitstellt.

Beispielsweise ist alternativ oder ergänzend auch vorgesehen, dass die Steuereinheit die zweite Betriebsweise mit zumindest, gegenüber der erste Betriebsweise, zeitweise abgeflachter inspiratorischer Rampe (IR2, IR3) bereitstellt.
Erfindungsgemäß ist alternativ oder ergänzend auch vorgesehen, dass die Steuereinheit die zweite Betriebsweise mit zumindest, gegenüber dem Niveau der erste Betriebsweise, zeitweise weniger sensitivem inspiratorischem Trigger (IT2, IT3) bereitstellt.
Erfindungsgemäß ist alternativ oder ergänzend auch vorgesehen, dass die Steuereinheit die zweite Betriebsweise mit zumindest, gegenüber dem Niveau der erste Betriebsweise, zeitweise sensitiverem exspiratorischem Trigger (ET2, ET3) bereitstellt.
Erfindungsgemäß ist alternativ oder ergänzend auch vorgesehen, dass die Steuereinheit die zweite Betriebsweise mit zumindest, gegenüber der erste Betriebsweise, zeitweise abgeflachter exspiratorischer (ER2, ER3) Rampe bereitstellt.
Erfindungsgemäß ist alternativ oder ergänzend auch vorgesehen, dass die Steuereinheit die zweite Betriebsweise mit zumindest, gegenüber der erste Betriebsweise, zeitweiser abgesenkter oder deaktivierter Hintergrundfrequenz (HF2, HF3) bereitstellt.
Erfindungsgemäß ist alternativ oder ergänzend auch vorgesehen, dass die Steuereinheit eine erste Betriebsweise mit mandatorischer Beatmung und eine zweite Betriebsweise mit assistierter Beatmung bereitstellt.
Die Steuereinheit gibt beispielsweise auf Anwenderauswahl hin die zweite Betriebsweise zeitgesteuert für einen begrenzten Zeitraum vor.
Die zweite Betriebsweise kann zeitgesteuert vorgegeben werden und liegt zum Beispiel zwischen 5 Minuten und 240 Minuten, bevorzugt zwischen 10 Minuten und 180 Minuten. Die Zeit für die zweite Betriebsweise kann bevorzugt anwenderseitig über ein Userinterface vorgegeben werden. Nach Ablauf des Zeitraums wird die Beatmung erfindungsgemäß auf diesem niedrigen Unterstützungs-Niveau gehalten, bis die Beatmung vom Anwender ausgeschaltet wird. In einer alternativen Ausführung kann - ggf. bedarfsabhängig - die Beatmung auch wieder zur ersten Betriebsweise zurückkehren.

Die Steuereinheit stellt die zweite Betriebsweise mit einem abnehmenden Maß an mechanischer Unterstützung der Atemarbeit bereit, wobei das Maß an Unterstützung jeweils nach einer gewissen Zeit oder nach einer gewissen Anzahl von Atemzügen, schrittweise gesenkt wird.

Alternativ kann im Rahmen der zweiten Betriebsweise ein abnehmendes mit einem vorübergehend wieder ansteigendes Maß mechanischer Unterstützung gekoppelt werden. In dieser Ausführungsform wird typisch während der zweiten Betriebsweise der Grad der mech. Unterstützung im Mittel über der Zeit dennoch abgesenkt. Gemäß dieser Ausführungsform wird der Patient quasi intermittierend wieder an eine Rekrutierung und Belastung der Atemmuskulatur herangeführt.

Die Länge der Phasen mit ansteigender oder wieder abnehmender Atemunterstützung können in einer Ausführung über einstellbare Zeiten oder eine einstellbare Anzahl von Atemzügen pro Phase festgelegt werden.

Beipielsweise kann eine Anpassung/Reduzierung des Grades der Atemunterstützung oder der Wechsel zwischen abnehmender Atemunterstützung und wieder zunehmender Unterstützung auf patientenseitigen Signalen basieren. Diese Signale können sein:
a) die Fluss- oder Drucksignale aus der Beatmung
b) Signale aus einer Pulsoxymetrie (Sauerstoffsättigung, Puls)
c) optional Signale aus einer CO2 Messung (transkutan oder endtidal im Atemgas).
zu a) Aus Fluss- und/oder Drucksignalen werden patientenseitige Einatmungen erkannt. Aus der Häufigkeit der Einatmungen (Atemfrequenz) kann ein Maß für den Lufthunger des Patienten abgeleitet werden. Ebenso ist es möglich, aus der Stärke des patientenseitig generierten Fluss-Signales ein Maß für den Lufthunger abzuleiten.
zu b) Aus pulsoxymetrischen Daten kann das Maß der Ventilation nicht ideal bestimmt werden, da in der Regel keine Informationen über CO2-Werte vorhanden sind. Dennoch können fallende SpO2 Werte oder eine steigende Pulsfrequenz ein Indiz für mehr Ventilationsbedarf, Lufthunger und/oder daraus resultierendem Stress sein.
zu c) CO2-Signale repräsentieren direkt das Maß der Ventilation. Basierend auf CO2 Werten lässt sich idealerweise abschätzen, inwieweit der Patient bei abnehmender Unterstützung durch das Beatmungsgerät die Ventilation wieder durch vermehrte Spontanatmung ausgleichen kann. Eine Anpassung der Atemunterstützung basierend auf CO2-Signalen kann insbesondere im Rahmen einer Beatmungs-Titration eingesetzt werden.

Erfindungsgemäß ist angedacht, dass diese Informationen der Steuereinheit zur Verfügung gestellt werden, die - basierend auf entsprechenden Algorithmen - Entscheidungen zum Erhöhung, konstant halten oder Absenken der Atmungsunterstützung ableitet.

Die Steuereinheit kann die zweite Betriebsweise, also das Modulieren (Senken, konstant halten oder zwischenzeitlich wieder Anheben) der Atmungsunterstützung, in grundsätzlich verschiedenen Weisen ausführen:
Dies kann beispielsweise in Form eines schrittweisen oder kontinuierlichen Absenkens des inspiratorischen Druckniveaus (IPAP) geschehen. Das insp. Druckniveau kann dabei in Schrittweiten bis 10 hPa pro Schritt reduziert werden. Erfindungsgemäß wird eine Schrittweite von 0,1 hPa bis 5 hPa pro Schritt bevorzugt.

Erfindungsgemäß kann dies auch in Form eines schrittweisen oder kontinuierlichen Absenkens des exspiratorischen Druckniveaus (EPAP) geschehen.
Der IPAP wird erfindungsgemäß schneller abgesenkt als der EPAP, so dass die Druckdifferenz (insp. Druckunterstützung) insgesamt abnimmt.

Die Steuereinheit kann die zweite Betriebsweise alternativ mit zeitweisen Phasen konstanten Druckes (CPAP-Phasen) bereitstellen, so dass die Atmungsunterstützung phasenweise vollständig entfällt. Durch einen CPAP Druck werden lediglich die oberen Atemwege sowie die kleinen Atemwege tendenziell offen gehalten werden. Auch sind bei einem CPAP Druck das endexspiratorische Lungenvolumen und die Gasaustauschfläche größer als bei Spontanatmung, so dass auch in den CPAP-Phasen trotz fehlender mechanischer Unterstützung der Einatmung ein positiver Effekt auf Lunge und/oder Atemwege erhalten bleibt.
Die Steuereinheit kann schließlich die in der zweiten Betriebsweise Beatmungshübe mit zumindest zeitweise verkürzter Inspirationszeit bereitstellen. Die Verkürzung der Inspirationszeit kann dabei ebenfalls über mehrere Schritte stattfinden. Erfindungsgemäß ist daran gedacht, dies über einen empfindlicher werdenden Exspirationstrigger zu realisieren, der - abhängig vom Flussverlauf - früher während der Einatmung auf das exsp. Druckniveau umschaltet. Die Inspirationszeit kann alternativ auch zeitgesteuert stattfinden, wobei die Steuereinheit eine Reduktion der vorgesehenen Inspirationszeit vornimmt.
Die Steuereinheit kann die zweite Betriebsweise ebenfalls mit zumindest zeitweise abgeflachter inspiratorischer Rampe bereitstellen, so dass sich die mechanische Unterstützung während der Einatmung langsamer aufbaut. Typischer Einatmungsdruckrampen liegen bei Werten größer 60 hPa/s, besser sogar größer als 100 hPa/s. Während der zweiten Phase ist erfindungsgemäß an Reduktion der Druckrampen kleiner als 60 hPa/s gedacht, wobei die Druckrampen auf Werte bis unter 20 hPa/s fallen können. In Grenzfällen wird das IPAP-Druckniveau gerade noch vor Umschaltung auf das exsp. Druckniveau erreicht, eine sonst beatmungstypische Druck-Plateauphase ausbleibt. Diese Ausführung kann auch mit der Reduktion des IPAP Druckes kombiniert werden.

Die Steuereinheit kann erfindungsgemäß die zweite Betriebsweise auch mit zumindest zeitweise weniger sensitivem inspiratorischem Trigger bereitstellen, so dass sich zwischen patientenseitiger Einatmung und mechanischer Unterstützung durch das Beatmungsgerät eine größere Verzögerungszeit einstellt

Die Steuereinheit kann ergänzend die zweite Betriebsweise mit zumindest zeitweise abgeflachter exspiratorischer Rampe bereitstellen, so dass die mechanische Unterstützung noch während der Einatmung früher, jedoch auf angenehme Weise, reduziert wird. Die angedachten exsp. Druckrampen können in der zweiten Betriebsweise bis auf Werte unter 10 hPa/s sinken. In Grenzfällen beginnt der Übergang auf die exsp. Rampe unmittelbar nach Erreichen des IPAP-Druckniveaus, womit eine sonst beatmungstypische Druck-Plateauphase ebenfalls ausbleibt. Auch diese Ausführungsform kann mit der Reduktion des IPAP Druckes kombiniert werden.

Es ist ebenfalls daran gedacht, dass die Steuereinheit die zweite Betriebsweise mit zumindest zeitweiser abgesenkter oder deaktivierter Hintergrundfrequenz bereitstellt, so dass zur mechanischen Unterstützung immer patientenseitige Einatembemühungen erforderlich sind. Mit dieser Ausführungsform wird besonders anschaulich, dass der Patient z.B. nach einer mandatorischen Beatmung während der ersten Betriebsweise nun während der zweiten Betriebsweise wieder an die - wenn auch unterstützte - Eigenatmung herangeführt wird.

Bei einer Modus-Umschaltung von einem T-Modus in einen ST-Modus oder aPCV-Modus, oder von einem ST-Modus in einen S-Modus wird der Beginn des Softstopps aktiviert.

Bevorzugt wird die Zeit des Softstopps nicht mit in die Therapiestatistik des Gerätes einberechnet wie Nutzungszeit, Volumenstatistik, Frequenzstatistik.

Die Funktion kann am Gerät nach Authentifizierung als Experten-User für den Patienten freigegeben oder gesperrt oder parametriert werden.

In einer alternativen Ausführung wird keine feste Zeit für die zweite Betriebsweise eingestellt werden, sondern das Gerät wartet nach jeder Absenkstufe der Atemunterstützung auf eine Bestätigung für die nächste Absenkung. Bestätigung kann sein: Benutzer-Eingabe oder Erreichen eines Zielwertes für die eigene Atemarbeit des Patienten, z. B. Erreichen eines bestimmten Volumen, einer Spontanatemfrequenz etc.

Erfindungsgemäß ist ein Feedback an den Patienten über das Display vorgesehen, ob und wie er die Zielwerte erreicht hat.

Alternativ kann die Eingabe einer subjektiven Bewertung der Softstopp-Geschwindigkeit durch den Patient erfolgen z. B. "zu langsam", "zu schnell", und daraufhin erfolgt automatisch eine Änderung einer Softstopp-Funktion, wie der Rampengeschwindigkeit, durch das Gerät.

Über die Messung einer Atemanstrengung oder SpO2 oder ptCO2 oder der autonomen Regulation des Körpers kann eine anfängliche Titration einer Softstopp-Geschwindigkeit erfolgen.

Weitere Therapiefunktionen werden während des Softstopp ebenfalls reduziert, entweder stufenförmig oder abhängig von Therapiedruck bzw. Atemfrequenz: Befeuchterleistung, O2-Einleitung, Erwärmung des Schlauches, Stärke einer autom. Druckregulation, z.B. Zielvolumensteuerung, auto-EPAP.

Das Gerät kann auch mit mehreren Beatmungsmodi programmiert werden. Dabei kann von jedem Beatmungsmodus auf den Softstopp umgeschaltet werden. Bevorzugt hängen dabei die Startwerte des Softstopps von den zuletzt eingestellten Beatmungsparametern des zuletzt eingestellten Beatmungsmodus ab, z. B. IPAP, EPAP, Frequenz, Triggersensitivität, inspiratorische Rampe, exspiratorische Rampe.

Das Beatmungsgerät kann mit mindestens einem automatischen Beatmungsmodus programmiert werden, bei dem EPAP und/oder IPAP und/oder Frequenz automatisch gesteuert werden. Bevorzugt hängen dabei die Startwerte des Softstopps von den zuletzt erreichten Beatmungsparametern des automatischen Beatmungsmodus ab, z. B. IPAP, EPAP, Frequenz, Triggersensitivität, inspiratorische Rampe, exspiratorische Rampe. Gehört zur ersten Betriebsweise ein mandatorischer Modus mit fester Atemfrequenz (Bsp.: PCV,T) kann in der zweiten Betriebsweise auch auf einen unterstützenden Modus gewechselt werden (Bsp.: PSV, aPCV, ST, S). Hierbei muss der beatmete Patient durch das Triggern des Gerätes auch dann wieder leichte Atemarbeit leisten, wenn die inspiratorische Druckunterstützung nicht vermindert wird.

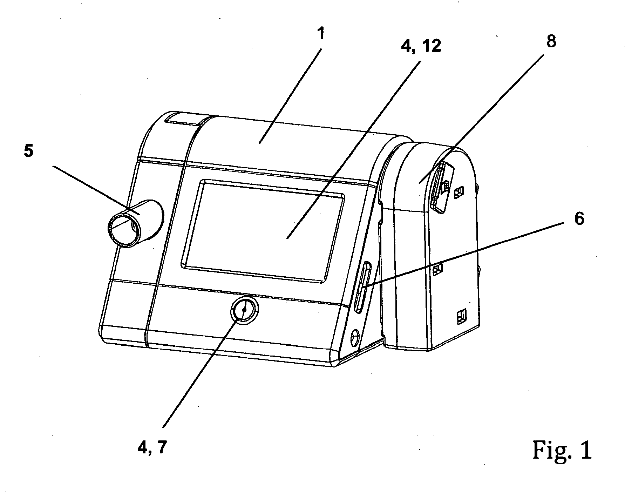

Fig. 1 zeigt ein Beatmungsgerät (1). Das Beatmungsgerät (1) ist mit einer Anzeigeeinrichtung (12), mindestens einem Bedienelement (7) sowie einem Schlauchanschluss (5) versehen.

Der Schlauchanschluss (5) dient typischerweise zur Verbindung mit einem nicht dargestellten Atemgasschlauch, der im Bereich seiner dem Schlauchanschluss (5) abgewandten Ausdehnung mit einer ebenfalls nicht dargestellten Atemmaske verbunden ist. Die nicht dargestellte Atemmaske dient zur Atemgasversorgung eines ebenfalls nicht dargestellten Patienten. Im Bereich des Schlauchanschlusses kann optional ein (nicht dargestellter) elektrischer Anschluss zur Beheizung eines Atemschlauches angebracht sein.

Das Beatmungsgerät (1) ist typischerweise mit mindestens einer Kommunikationsschnittstelle (6) versehen. Die Schnittstelle (6) ist hier in einem Seitenbereich angeordnet. Die Schnittstelle (6) ist zur Horizontalen geneigt und befindet sich in räumlicher Nähe zum Display (12). Bevorzugt weist das Beatmungsgerät zudem eine Schnittstelle (6') auf die der Kopplung von Modulen (8) dient.

Bei dem dargestellten Ausführungsbeispiel ist das Beatmungsgerät (1) nicht mit einem Atemgasbefeuchter (7) gekoppelt. Das Beatmungsgerät (1) kann optional sowohl mit als auch ohne den Atemgasbefeuchter (7) betrieben werden.

Gemäß der im Fig. 1 dargestellten Ausführungsform weist das Gehäuse des Beatmungsgerätes (1) in einer senkrechten Schnittebene eine dreieckartige Querschnittfläche auf.

Gemäß der Ausführungsform in Fig. 1 können an das Beatmungsgerät (1) bedarfsabhängig Zusatzelemente (8) angekoppelt werden, die mindestens eine weitere Gerätefunktion bereitstellen. Das Display (12) ist hier als Benutzerschnittstelle, beispielsweise als Touchscreen, des Beatmungsgerätes (1) ausgeführt. Im Bereich des Displays (12) sind mehrere Eingabetasten vorgesehen, die zum Empfangen einer Benutzereingabe konfiguriert sind. Das Display (12) ist schaltlogisch mit den Tasten verbunden und weist mehrere Informationsfelder auf wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben. Die Eingabetasten sind berührempfindliche Flächen im Bereich des Displays (12) Erfindungsgemäß ist mindestens ein weiteres Bedienelement (3)vorgesehen. Dieses Bedienelement (7) ist mechanisch bedienbar und unterscheidet sich somit von den Berührflächen auf dem Display. Erfindungsgemäß wird das Beatmungsgerät über das Bedienelement (3) ein- und ausgeschaltet, 'sodass eine grundlegende, patientengerechte Therapie mittels lediglich einer Taste aktiviert werden kann. Dazu ist das Bedienelement (7) schaltlogisch mit dem Gebläsemotor verbunden und aktiviert diesen, sowie den Speicher um hinterlegte Therapiedaten, wie Druckwerte, abzurufen und anzuwenden. Erfindungsgemäß wird die Steuereinheit (2) über das Bedienelement (4) aktiviert, sodass die zweite Betriebsweise (30) abgerufen wird. Dazu ist das Bedienelement (4) schaltlogisch mit dem Gebläsemotor verbunden und aktiviert diesen, sowie den Speicher um hinterlegte Parameter für die zweite Betriebsweise, wie beispielsweise Zeitdauer, Druckwerte, inspiratorische oder exspiratorische Rampe abzurufen und anzuwenden.

Ergänzend kann über die Eingabetasten eine Anpassung einzelner Werte, wie Druckwerte, erfolgen. Eine Bedienung über die Eingabetasten (4) des Touchscreens (12) ist erfindungsgemäß jedoch nicht notwendig, um die Therapie zu starten.

In einer Ausführungsform ist angedacht, dass der Taster (3 oder 4), über den das Gerät aktiviert und deaktiviert wird, in folgender Art den Betrieb des steuert: Ist die zweite Betriebsweise im Gerät vom Arzt freigeschaltet, dann reagiert das Gerät unter der Therapie in der ersten Betriebsweise so, dass die einmalige Bedienung des (Ein-/Aus-) Tasters nicht die Therapie beendet, sondern das die Beatmung in die zweite Betriebsweise übergeht. Erst ein erneutes Drücken des Ein-/Aus Tasters führt in dieser Ausführung zum Beenden der Beatmung.

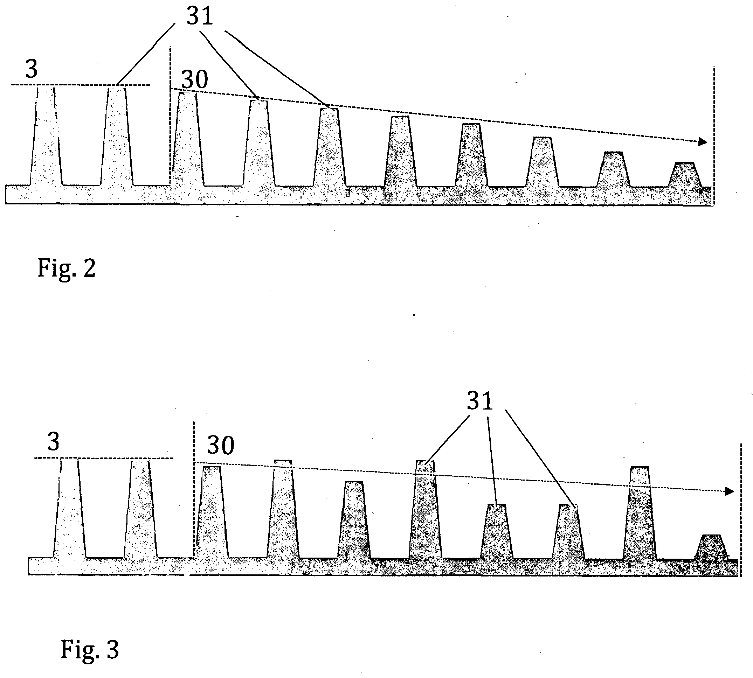

Fig. 2 zeigt den zeitlichen Verlauf der beiden Betriebsweisen. Das Beatmungsgerät (1) mit der Steuereinheit (2) gibt eine erste Betriebsweise (3), mit einem hohen Maß an Unterstützung der Atemarbeit vor. Nach Aktivierung über ein Auswahlmittel (4), stellt die Steuereinheit eine zweite Betriebsweise (30), mit einem geringeren Maß an Unterstützung der Atemarbeit, bereit. Hier sinkt das Maß der Unterstützung dadurch, dass mit jedem Atemzyklus ein geringerer IPAP-Druck (31) vorgegeben wird.

Fig. 3 zeigt einen alternativen zeitlichen Verlauf der beiden Betriebsweisen. Das Beatmungsgerät (1) mit der Steuereinheit (2) gibt eine erste Betriebsweise (3), mit einem hohen Maß an Unterstützung der Atemarbeit vor. Nach Aktivierung über ein Auswahlmittel (4), stellt die Steuereinheit eine zweite Betriebsweise (30), mit einem geringeren Maß an Unterstützung der Atemarbeit, bereit. Hier sinkt das Maß der Unterstützung dadurch, dass mit jedem Atemzyklus ein anderer IPAP-Druck (31) vorgegeben wird, wobei nach einem geringeren Druck auch wieder ein erhöhter Druck vorgegeben werden kann, jedoch der Druck im Mittel über Zeit sinkt.

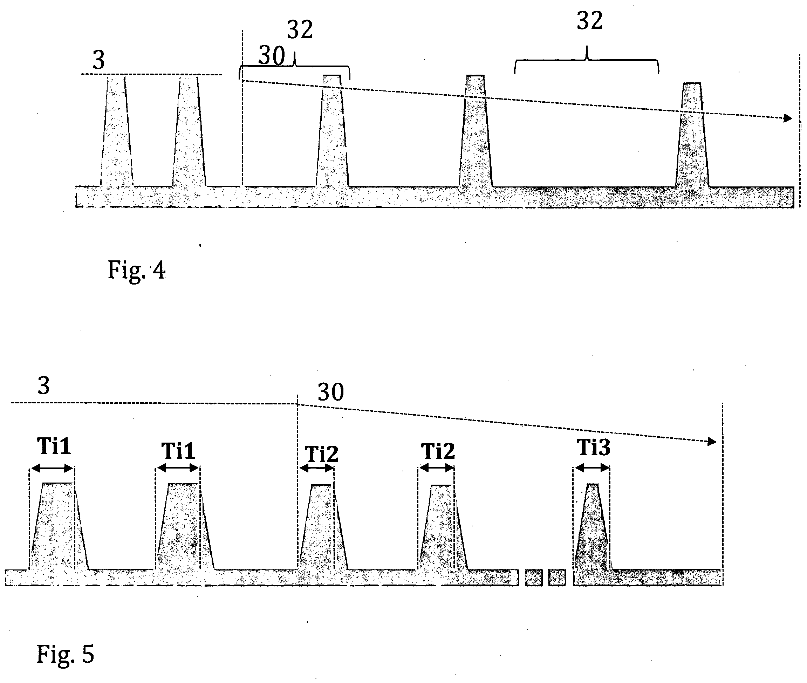

Fig. 4 zeigt einen anderen alternativen zeitlichen Verlauf der beiden Betriebsweisen. Das Beatmungsgerät (1) mit der Steuereinheit (2) gibt eine erste Betriebsweise (3), mit einem hohen Maß an Unterstützung der Atemarbeit vor. Nach Aktivierung über ein Auswahlmittel (4), stellt die Steuereinheit eine zweite Betriebsweise (30), mit einem geringeren Maß an Unterstützung der Atemarbeit, bereit. Hier sinkt das Maß der Unterstützung dadurch, dass für einige Atemzyklen lediglich ein konstanter Druck (32), und kein IPAP, vorgegeben wird und somit die Unterstützung geringer ausfällt.

Fig. 5 zeigt einen nochmals anderen alternativen zeitlichen Verlauf der beiden Betriebsweisen. Das Beatmungsgerät (1) mit der Steuereinheit (2) gibt eine erste Betriebsweise (3), mit einem hohen Maß an Unterstützung der Atemarbeit, mit einer Inspirationszeit (Ti1) vor. Nach Aktivierung über ein Auswahlmittel (4), stellt die Steuereinheit eine zweite Betriebsweise (30), mit einem geringeren Maß an Unterstützung der Atemarbeit, bereit. Hier sinkt das Maß der Unterstützung dadurch, dass die Steuereinheit (2) die zweite Betriebsweise (30) mit zumindest zeitweise verkürzter Inspirationszeit (Ti2, Ti3) oder weiter abnehmender Inspirationszeit bereitstellt.

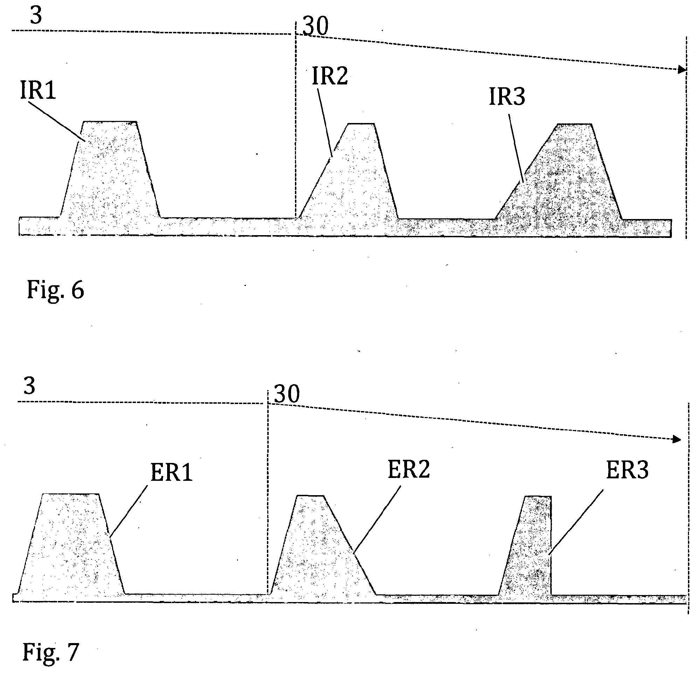

Fig. 6 zeigt einen weiteren alternativen zeitlichen Verlauf der beiden Betriebsweisen. Das Beatmungsgerät (1) mit der Steuereinheit (2) gibt eine erste Betriebsweise (3), mit einem hohen Maß an Unterstützung der Atemarbeit, mit einer Inspirationszeit (Ti1) vor. Nach Aktivierung über ein Auswahlmittel (4), stellt die Steuereinheit eine zweite Betriebsweise (30), mit einem geringeren Maß an Unterstützung der Atemarbeit, bereit. Hier sinkt das Maß der Unterstützung dadurch, dass die Steuereinheit (2) die zweite Betriebsweise (30) mit zumindest zeitweise verkürzter Inspirationszeit (Ti2, Ti3) oder weiter abnehmender Inspirationszeit bereitstellt.

Die in den Fig. 2 bis 6 abgebildeten Druckverläufe zeigen nur sehr einfach und beispielhaft, aber nicht repräsentativ, über wie viele Atemzüge oder über welche Zeit während der zweiten Betriebsweise jeweils ein bestimmtes Unterstützungsniveau bei-behalten wird oder wie häufig der Grad der Unterstützung innerhalb der zweiten Betriebsweise angepasst wird.

## Patentansprüche

1. Beatmungsgerät (1) mit einer Steuereinheit (2) die so konfiguriert ist, dass sie eine erste Betriebsweise (3), mit einem IPAP und einem EPAP, und mit einem hohen Maß an Unterstützung der Atemarbeit bereitstellt, wobei die Steuereinheit (2) so konfiguriet ist, dass nach Aktivierung über ein Auswahlmittel (4), eine zweite Betriebsweise ( 30), mit einem geringeren Maß an Unterstützung der Atemarbeit, bereitgestellt wird, wobei die Steuereinheit so konfiguriert ist, dass in der zweiten Betriebsweise der IPAP schneller abgesenkt wird als der EPAP, sodass die Druckdifferenz (inspiratorische Druckunterstützung) insgesamt abnimmt,
**dadurch gekennzeichnet, dass** die Steuereinheit (2) auf Anwenderauswahl hin, die zweite Betriebsweise zeitgesteuert für einen begrenzten Zeitraum vorgibt, wobei die Steuereinheit (2) die Beatmung, nach Ablauf des Zeitraums der zweiten Betriebsweise, auf diesem niedrigen Unterstützungs-Niveau hält, bis die Beatmung vom Anwender ausgeschaltet wird.

2. Beatmungsgerät nach zumindest einem der vorhergehenden Ansprüche
**dadurch gekennzeichnet, dass** die Steuereinheit (2) während der zweiten Betriebsweise (30) das Maß der Unterstützung der Atemarbeit schrittweise anpasst.

3. Beatmungsgerät nach zumindest einem der vorhergehenden Ansprüche
**dadurch gekennzeichnet, dass** während der zweiten Betriebsweise (30) der Wechsel zwischen Phasen mit unterschiedlicher Unterstützung der Atemarbeit auf Signalen der Beatmung beruht.

4. Beatmungsgerät nach zumindest einem der vorhergehenden Ansprüche **dadurch gekennzeichnet, dass** während der zweiten Betriebsweise (30) der Wechsel zwischen Phasen mit unterschiedlicher Unterstützung der Atemarbeit auf Signalen aus einer Pulsoxymetrie und/oder auf CO2-Signalen beruht.

5. Beatmungsgerät nach zumindest einem der vorhergehenden Ansprüche **dadurch gekennzeichnet, dass** die Steuereinheit (2) die zweite Betriebsweise (30) mit zumindest zeitweise verkürzter Inspirationszeit (Ti2, Ti3) bereitstellt.

6. Beatmungsgerät nach zumindest einem der vorhergehenden Ansprüche **dadurch gekennzeichnet, dass** die Steuereinheit (2) die zweite Betriebsweise (30) mit zumindest zeitweise weniger sensitivem inspiratorischem Trigger (IT2, IT3) bereitstellt.

7. Beatmungsgerät nach zumindest einem der vorhergehenden Ansprüche **dadurch gekennzeichnet, dass** die Steuereinheit (2) die zweite Betriebsweise (30) mit zumindest zeitweise sensitiverem exspiratorischem Trigger (ET2, ET3) bereitstellt.

8. Beatmungsgerät nach zumindest einem der vorhergehenden Ansprüche **dadurch gekennzeichnet, dass** die Steuereinheit (2) die zweite Betriebsweise (30) mit zumindest zeitweise abgeflachter exspiratorischer (ER2, ER3) Rampe bereitstellt.

9. Beatmungsgerät nach zumindest einem der vorhergehenden Ansprüche **dadurch gekennzeichnet, dass** die Steuereinheit (2) die zweite Betriebsweise (30) mit zumindest zeitweiser abgesenkter oder deaktivierter Hintergrundfrequenz (HF2, HF3) bereitstellt.

10. Beatmungsgerät nach zumindest einem der vorhergehenden Ansprüche **dadurch gekennzeichnet, dass** die Steuereinheit eine erste Betriebsweise mit mandatorischer Beatmung und eine zweite Betriebsweise mit assistierter Beatmung bereitstellt.

## Claims

1. A ventilator (1) having a control unit (2) which is configured such that a first mode (3) is provided that has an IPAP and an EPAP and a high degree of support of respiratory effort, wherein the control unit (2) is configured such that a second mode (30) is provided that has a lesser degree of support of respiratory effort after being activated by a selection means (4), wherein the control unit is configured such that in the second mode, the IPAP is reduced faster than the EPAP, such that the pressure differential (inspiration pressure support) decreases overall, **characterized in that** in response to the user's selection, the control unit (2) dictates the second mode in a time-controlled manner for a limited period, wherein after cessation of the period of the second mode, the control unit (2) maintains ventilation at this low support level until ventilation is turned off by the user.

2. The ventilator according to at least one of the preceding claims, **characterized in that** the control unit (2) adapts the degree of support of respiratory effort step by step during the second mode (30).

3. The ventilator according to at least one of the preceding claims, **characterized in that** during the second mode (30), the switch between phases with different support of respiratory effort is based on signals from ventilation.

4. The ventilator according to at least one of the preceding claims, **characterized in that** during the second mode (30), the switch between phases with different support of respiratory effort is based on signals from pulse oximetry and/or CO2 signals.

5. The ventilator according to at least one of the preceding claims, **characterized in that** the control unit (2) provides the second mode (30) having an at least temporarily shorter inspiration time (Ti2, Ti3) .

6. The ventilator according to at least one of the preceding claims, **characterized in that** the control unit (2) provides the second mode (30) having an at least temporarily less sensitive inspiration trigger (IT2, IT3).

7. The ventilator according to at least one of the preceding claims, **characterized in that** the control unit (2) provides the second mode (30) having an at least temporarily more sensitive expiration trigger (ET2, ET3).

8. The ventilator according to at least one of the preceding claims, **characterized in that** the control unit (2) provides the second mode (30) having an at least temporarily flat expiration (ER2, ER3) ramp.

9. The ventilator according to at least one of the preceding claims, **characterized in that** the control unit (2) provides the second mode (30) having an at least temporarily reduced or deactivated background frequency (HF2, HF3).

10. The ventilator according to at least one of the preceding claims, **characterized in that** the control unit provides a first mode having mandatory ventilation, and a second mode having assisted ventilation

## Revendications

1. Respirateur artificiel (1) doté d'une unité de commande (2) qui est configurée de manière à ce qu'elle mette à disposition un premier mode de fonctionnement (3), présentant une pression inspiratoire IPAP et expiratoire EPAP positive dans les voies aériennes, et procurant un haut niveau d'assistance respiratoire, dans lequel l'unité de commande (2) est configurée de manière à ce qu'un second mode de fonctionnement (30) procurant un moindre niveau d'assistance respiratoire est mis à disposition après l'activation via un moyen de sélection (4), dans lequel l'unité de commande est configurée de manière à ce que l'IPAP baisse plus vite que l'EPAP dans le second mode de fonctionnement, si bien que la différence de pression (aide inspiratoire) diminue globalement, **caractérisé en ce que** l'unité de commande (2) prédéfinit le second mode de fonctionnement au choix de l'utilisateur en le programmant dans le temps durant une période limitée, dans lequel l'unité de commande (2) maintient la respiration artificielle sur ce faible niveau d'assistance à la fin de la période du second mode de fonctionnement, jusqu'à ce que la respiration artificielle soit arrêtée par l'utilisateur.

2. Respirateur artificiel selon au moins une des revendications précédentes, **caractérisé en ce que** l'unité de commande (2) adapte progressivement le niveau d'assistance respiratoire durant le second mode de fonctionnement (30).

3. Respirateur artificiel selon au moins une des revendications précédentes, **caractérisé en ce que** le passage entre les phases présentant une assistance respiratoire différente repose sur les signaux de la respiration artificielle durant le second mode de fonctionnement (30).

4. Respirateur artificiel selon au moins une des revendications précédentes, **caractérisé en ce que** le passage entre les phases présentant une assistance respiratoire différente repose sur les signaux d'une pulsoxymétrie et/ou sur les signaux de CO2 durant le second mode de fonctionnement (30).

5. Respirateur artificiel selon au moins une des revendications précédentes, **caractérisé en ce que** l'unité de commande (2) met à disposition le second mode de fonctionnement (30) avec un temps d'inspiration raccourci au moins par intermittence (Ti2, Ti3).

6. Respirateur artificiel selon au moins une des revendications précédentes, **caractérisé en ce que** l'unité de commande (2) met à disposition le second mode de fonctionnement (30) avec un déclencheur inspiratoire moins sensible au moins par intermittence (IT2, IT3).

7. Respirateur artificiel selon au moins une des revendications précédentes, **caractérisé en ce que** l'unité de commande (2) met à disposition le second mode de fonctionnement (30) avec un déclencheur expiratoire plus sensible au moins par intermittence (ET2, ET3).

8. Respirateur artificiel selon au moins une des revendications précédentes, **caractérisé en ce que** l'unité de commande (2) met à disposition le second mode de fonctionnement (30) avec une rampe expiratoire aplatie au moins par intermittence (ER2, ER3).

9. Respirateur artificiel selon au moins une des revendications précédentes, **caractérisé en ce que** l'unité de commande (2) met à disposition le second mode de fonctionnement (30) avec une fréquence de fond diminuée ou désactivée au moins par intermittence (HF2, HF3).

10. Respirateur artificiel selon au moins une des revendications précédentes, **caractérisé en ce que** l'unité de commande met à disposition un premier mode de fonctionnement présentant une respiration artificielle imposée et un second mode de fonctionnement présentant une respiration artificielle assistée.
